# EUROPEAN PATENT APPLICATION

(11) **EP 0 875 224 A1**
(43) Date of publication of application: **04.11.1998**
(21) Application number: 97107002.4
(22) Date of filing: 28.04.1997
(51) Int. Cl.: A61F 13/15

(54) **Absorbent article which includes superabsorbent material located in discrete pockets and manufacturing process**

(71) Applicant: Cidieffe S.r.l., 20149 Milano (IT)
(72) Inventor: Cerutti, Gianvito, 20149 Milan (IT)
(74) Representative: Robba, Pierpaolo

(57) **Abstract**

A layered absorbent structure is disclosed comprising two sheets (1, 3) of materials permeable to fluids between which an absorbent core (2) is sandwiched, such core comprising a sheet (10) of a material embossed with reliefs (4) so as to form recesses (6) separate from each other and projecting from at least one face thereof. The embossed sheet (10) is bonded to said sheet (1) at least along peripheral portion (5A) surrounding the base of the said recesses (6), so as to form tridimensional separate and closed containers (8) containing an absorbent material, around said containers (8) there being formed a network of channels (7) allowing for a quick flow of the liquid for bypassing the saturated containers.

There are further disclosed processes for manufacturing the layered structures and/or absorbent articles incorporating said structures as absorbent cores.

## Description

The present invention is concerned with layered absorbent products and articles of the type comprising two or more sheets of material, such as for example a support or backing sheet, a distribution sheet and a containment sheet for the storage of the absorbed liquid or fluid. In the present disclosure the terms liquid and fluid will be interchangeably used to indicate materials that are neither solid nor gaseous.

More particularly the invention relates to an absorbent structure formed by two or more layers and provided with a layer having characteristics of absorbtion and distribution of the liquid.

A layer performing both the functions of acquisition and distribution of the fluid to be absorbed is generally known as an ADL layer (Acquisition and Distribution Layer). Traditionally an ADL or Air through bonding layer has been defined as a layer of absorbent material capable of getting the liquid that has passed through a topsheet and spreading it very quickly into the body of the whole ADL layer ADL thus allowing a complete exploitation of the absorbing core surface.

The acquisition of the liquid and the quick distribution thereof can be achieved through a tridimensional structure only, i.e. a structure having a thickness that is not negligible with respect to the size of the absorbent article.

Preferably, but not exclusively, the structure according to the invention forms the absorbent core of finished articles that are - again preferably - disposable, i.e. to be disposed of after being used.

The invention is further concerned with a process for manufacturing such a structure by embossing a sheet that can be either of a woven fabric or a nonwoven material or a perforated plastic film, into a structure having a desired shape and configuration.

For a better understanding of the invention, as herein used, the expression "absorbent article" indicates an absorbent product, finished and ready to be used, for example a sanitary napkin, a disposable diaper, a drawsheet for incontinents or animals, or a properly sized and shaped absorbent sheet to be inserted in a tray for storing foods, or other absorbent devices. On the other hand, the expressions "absorbent core" and "ADL" indicate single-layer or multilayer products, in case composite products, used as components in the manufacturing of absorbent articles.

In the manufacture of absorbent articles there is a growing need to use ADL components, such need deriving on one hand from the spreading use of powders and fibres of superabsorbent materials (Super Absorbent Polymers or SAP) in the absorbent core, and on the other hand by need - much more deeply felt with respect to the past - of realizing very thin absorbent articles.

These absorbent articles, while thinner than the conventional ones, must exhibit an equal or better absorbency performance than that of the traditional articles with a larger thickness. This lead to using powders and fibers of superabsorbent materials, also called SAP, in the structure of the absorbent core, and to reduce the amount of bulky materials, such as for example the so-called "fluff".

On the other hand the acquisition and the retaining of the liquid by these thin absorbent cores is not immediate because of the characteristics of the SAP materials that in presence of a large mass of liquid, tend to form a gel that in turn hinders or even blocks the further diffusion (spreading) of the liquid to be absorbed. In accordance with the prior art, to overcome this shortcoming and prevent the possible risk of leaving the liquid in contact with the user's skin, a rather bulky (tridimensional) ADL layer is inserted between the absorbent core and the topsheet. Therefore, although ADL layers have been used for many years, in the finished absorbent article the ADL layer has become an important component and it has become extremely desirable to reduce its thickness.

To meet the above known requirements it has been suggested to use products of a nonwoven highloft fabric, formed for example by synthetic fibers (polyethylene, polyester, polypropylene), or by natural fibers bonded together with hot air, bonding agents, or mechanical means such as the needling process.

Other suggestions were directed to the use of ADL materials, formed by cellulosic fibers and/or polymer fibers, manufactured through a so-called air laid process, or through other known processes for forming nonwoven fabrics.

There are further known products obtained by combining or layering films of synthetic materials and fibers.

The products obtained through these techniques are in form of (substantially smooth) sheets having a density between 20 g/m² and 300 g/m², and a thickness variable from 1 to 30 mm, used either as a single layer or as a multilayer element, and working through small perforations (from the top to the bottom) and/or capillary structures created by a pattern of fibers inside the fabric only allowing for a limited distribution of the liquid through the layer.

Moreover these products are poorly satisfactory from the viewpoint of the combined features: for example the liquid distribution can be very good, but associated with a modest liquid acquisition, or viceversa. Additionally the manufacturing of the product becomes complex and therefore with unacceptable costs for disposable articles.

There are known several ADL absorbent structures with different constructions.

US-A-4 578 068 is concerned with a layered absorbent structure having a substantially uniform density and comprising two or more sheets of fibrous material between which a SAP material is disposed, said sheets being deformed together so as to form piramidal or conical projections. The SAPs form a discontinuous layer sandwiched between pairs of sheets, both deformed of which together, so that the SAP materials are not located inside the recesses forming the channels.

WO 9211830 and WO 9211831 provide for (at least) an ADL layer and an adjacent layer of absorbent material capable of gelling when absorbing liquids.

EP-A-0 613 671 provides for a layer - that can incorporate SAP materials - embossed or relief printed to form a pattern with portions having different densities to improve the liquid absorption and preventing a "gel blocking" situation.

WO 9507673 provides for a layer of absorbent material wherein a plurality of channels is embossed to improve the distribution of the fluid over an extended area.

US-A-4 840 692 and US-A-4 758 240 provide for channels formed by removing material from the core thickness.

FR-A-2 279 373 discloses a three-layer absorbent member, with the intermediate layer embossed with ridges, and the upper layer provided with holes for accelerating the distribution of the fluid.

It is an object of the present invention to realize an absorbent structure adapted to be used in an absorbent article, thai is capable to overcome the above limitations and shortcomings of the prior art. The invention is further concerned with a process for manufacturing such absorbent structure.

These objects are accomplished thanks to the characteristics recited in claims 1 and 8, whereas further advantageous characteristics are disclosed in the dependent claims.

The structure according to the invention provides for raised or relief portions - properly distribuited according to an embossing configuration or a predetermined pattern - that define recesses closed by a topsheet to form containers for absorbent substances, and substantially void channels surrounding the recesses and consenting the diffusion of the liquid through a flow that is substantially not hindered or in case facilitated, rather than through a process of absorption (this latter occurring substantially in the containers housing the absorbent material).

The sheet in which the recesses are formed is associated and bonded to at least another sheet of material permeable to the fluids, forming with the recesses of the tridimensional structure housings or containers that are closed and separated from each other by flat portions adjacent to other recesses or channels. These latter cavities or channels can be closed by an additional sheet. At least a number, and preferably all the closed containers, end in case the channels too, can be partially filled with materials of various type and for various functions, such materials being in form of powders, granules, fibers or the like. These materials comprise substances capable of absorbing the liquids or the odors, scented substances, or the like. Preferably the so-formed containers are partially filled with an absorbent material, and more preferably with materials of very high absorbency or SAP, for example in powder form. This way the structure of the invention accomplishes a twofold function: the cavities of the channels offer a distribution and diffusione property to the liquid to be absorbed, whereas the embossed portions containing the absorbent powders form the true absorbent core of the structure, and therefore of the final article.

The invention achieves an optimum use of the absorbent materials, particularly superabsorbent materials or SAP, since it allows the liquid to bypass the barrier constituted by the mass of gelled SAP material, thus passing through speces that are empy or anyhow capable to present a negligible obstacle to the liquid flow.

Moreover and more precisely, according to the invention it is incremented the diffusion capability of an ADL layer, when this latter is present in the structure or forms the embossed sheet.

The invention will now be disclosed with reference to preferred but non limiting embodiments, illustrated in the attached drawings in which:
Fig. 1 illustrates in a schematic and partial drawing an absorbent structure according to the invention;
Fig. 2 shows a more detailed cross-section of the structure of Fig. 1;
Fig. 3 is a bottom view of the absorbent structure of Fig. 2 with the topsheet 3 partially broken away;
Figs. 4 to 7 are partial schematic cross section views of further embodiments of structures according to the invention;
Fig. 8 schematically illustrates the relief printing of a sheet according to the invention;
Fig. 9 illustrates a second embodiment for relief printing a sheet;
Figs. 10 and 10A illustrate an embodiment of the process according to the invention; and
Figs. 11 and 11A illustrate a second embodiment of the process according to the invention.

In the drawings components that are equal or substantially equivalent are indicated by the same numeral references throughout all the Figures.

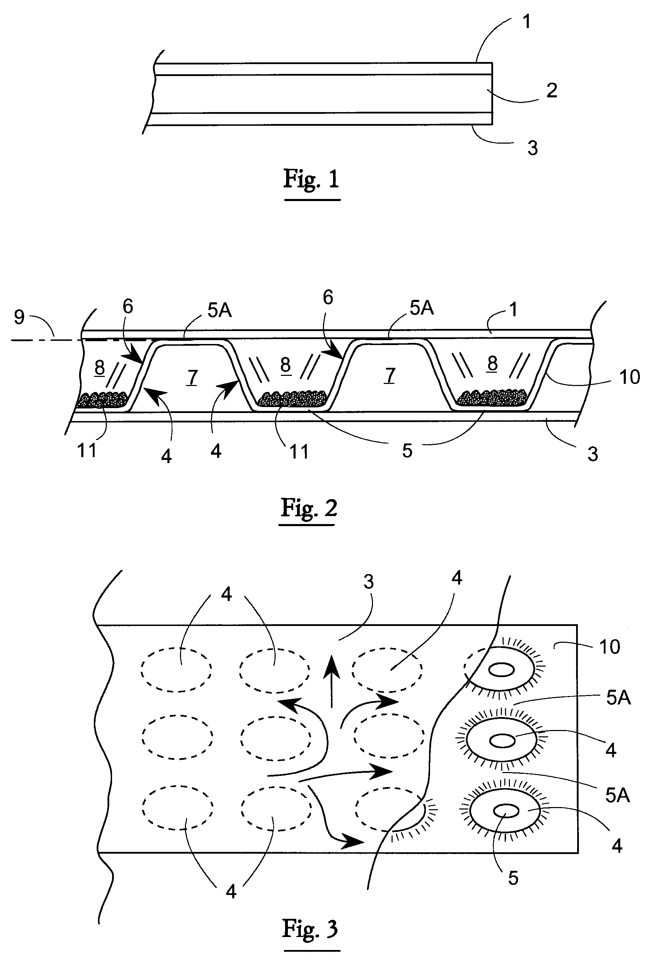

Firstly with reference to Fig. 1, an absorbent structure according to the invention comprises at least tre component layers, namely a first outer sheet or topsheet 1 of a material permeable to fluids, an absorbent core 2 and a second outer sheet or backsheet 3 of a material permeable to fluids. The layers forming such components or sheets are superimposed on each other and at least locally bonded together. The materials forming the sheets 1 and 2 are convenzional, for example a continuous nonwoven fabric and staple bonded, dry paper, plastic film, in case perforated, composite materials, layered composites, etc.

As better shown in Figs. 2 and 3, the absorbent structure according to the invention comprises at least a sheet 10 of a material that has been embossed or relief printed to form reliefs or prominences 4 so as to reach a three-dimensional configuration with respect to the original plane of the sheet (or base plane 9), and to form recesses 6 separate from each other. In the embodiment illustrated in Figures 2 and 3, the reliefs 4 project downwards from the original (undeformed) plane 9 (base plane) of the sheet 10 before it is embossed.

The sheets 1 and 3 are bonded to the tridimensional layer 10 by means of glueing, pasting or other known means, in corrispondence of the peripheral portions or areas 5A of the sheet 10 that have not been deformed - and there fore substantially lay in the base plane 9 - and in correspondence of the lower bases 5 of the recesses 6.

Preferably, as shown in the Figures 2 to 6, such reliefs projects from one face only of the baseplane 9, however the reliefs can extend from both sides of the original sheet 10, with equal or different heights.

The material of the sheet 10 can be formed, for example, by nonwoven short and continuous thermobonded fibers, dry laid in case with added fibers and polymers, composites and layered, plastic films with micro-perforations, etc..

For clarity sake, in the Figures the spacing between the reliefs 4 and the size of these latter are not scale drawn. Preferably the spacing between the recesses 6 defined by the embossed dai reliefs is variable as a function of the shape of the containers and their arrangement (aligned or offset). Preferably the base area of the containers is smaller than surrounding area.

This way it is obtained a tridimensional structure with closed and separate containers 8, defined between the reliefs 4 and the topsheet 1, and a network of channels 7 defined between the reliefs 5 and the backsheet 3, extending around the containers 8 and capable of performing a liquid distribution function.

The bottom view of Fig. 3 illustrates recesses 6 having an elongated shape, with a contact area 5A for joining to the topsheet 1 that extends around each container 8.

At least some, but preferably all the containers 8 contains a material 11 that fills them only partially as will be discussed later.

Such material can be in form of powders, granules, fibers, scales or other non-compact forms. Preferably such material is an absorbent material, and more preferably it is a superabsorbent material, i.e. a material having a very high absorbency. In this latter case, only a relatively small volume of the container is occupied by the superabsorbent material. More precisely, the amount of dry SAP material present in the containers 8 is selected to be contained within the containers after its gelling. In other words, the amount of powder is selected in such a manner that after being soaked, it swells to a volume smaller or equal to that of the container in which it is housed, thus preventing the gel from overflowing the container boundaries and come in contact with the channel surrounding the container, which would create a barrier to the flow of the liquid into the surrounding channels. As a general rule, this criterium applies also to the conventional absorbent materials.

When the containers 8 contain an absorbent or superabsorbent material, they absorb the liquid and perform a sealing function (by preventing the outcome of the gel), but not a complete ADL function (that not only provides for the acquisition but also for the distribution of the liquid). On the other hand the channels or cavities 7 adjacent the containers 8 perform a distribution function since they are void (as shown in the embodiment of Fig. 2), or in case partially filled with a material having a limited absorbency and capable of spreading the liquid. The liquids that reach these channels through capillarity are routed towards the adjacent container 8 that is less saturated and anyhow still exhibiting a residual capacity of absorbing and retaining the liquid. This effect is schematically shown in Fig. 3 by arrows indicating the paths of the liquid flow.

The network of cavities or channels 7 surrounding the containers 8 enhance the diffusion capability of an ADL layer by offering an unhindered passage to the liquid, or even an easier passage when the channels contain materials that improve the flow through a capillarity effect.

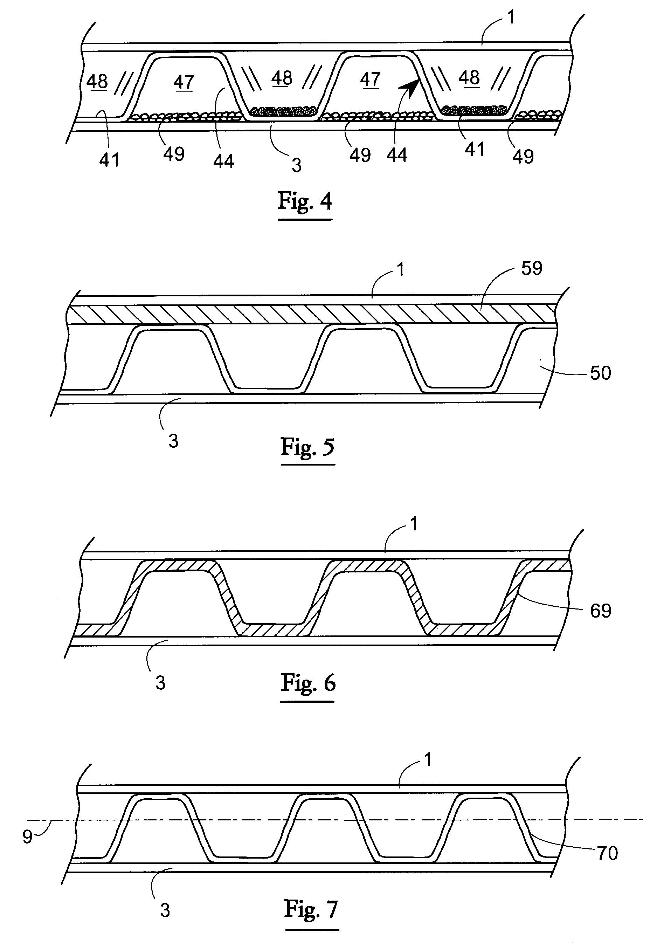

The embodiment shown in Fig. 4, provides for a topsheet 1 and a backsheet 3 between which there is sandwiched a sheet of material che has been subjected to a tridimensional embossing process or otherwise shaped to form a structure having raised portions or reliefs 44 that together with the topsheet 1 define closed containers 48. The cross section shape of the reliefs is for example square or rectangular.

In this embodiment, in the channels 47 formed between the outer walls of the reliefs 44 and the sheet 3, which channels surround the containers 48, a material 49 is provided that does not impede the flow of the fluid. Preferably this material occupies a fraction only of the available space and is formed e.g. by scented sustances, capable of absorbing the odours, or by fibers adapted to facilitate the transmission of the liquids through capillarity, or a mixture of these substances. When in the containers 48 there is present a traditional absorbent material (not a superabsorbent material), an absorbent material can be disposed in the channels 47 too, having an absorbency quite lower in respect of the material in the containers 48.

In the embodiment illustrated in the cross-section view of Fig. 5, the absorbent structure comprises two outer sheets 1 and 3, an ADL layer 59 of conventional construction in contact with one of the two, for example sheet 1, and a tridimensionally embossed sheet 50 disposed between the layer ADL and the other sheet. The layers are bonded to each other, for example by means of an adhesive. Although not shown in the Figure, absorbent materials or material with other properties can be present in the containers and in the channels formed in the absorbent structure of the invention.

In the embodiment shown in Fig. 6, the absorbent structure comprises two outer sheets 1 and 3, between which an ADL layer 69 of conventional type is sandwiched, the layer 69 being relief printed to form containers and channels according to the principles of the invention. Also in this construction absorbent materials or with other functions can be present in the containers and in the channels.

Fig. 7 schematically illustrates an embodiment in which a sheet 70 is relief printed to form recesses on both faces of the sheet with respect to the original plane 9, and the three-dimensional sheet 70 is sandwiched between two sheets 1 and 3.

In general, an absorbent or superabsorbent material is preferably placed within the containers whereas the channels are either empty or containing scented substances, odours-adsorbing substances, or fibers capable to spread the liquid by capillarity, or a mixture of the above substances. When the containers contain a conventional absorbent material, even an absorbent material having absorbency capabilities quite lower than that in the containers can be present in the channels.

Indicatively the density of the materials in the containers and/or the channels is comprised between 10 g/m² and 400 g/m².

The portions in relief can have a cross-section shape that is substantially rectangular or square, circular or elliptical, or with other shapes that have not been illustrated in the drawings. When the reliefs are formed as spherical caps, their diameter can be within 5 and 15 mm, and their height is preferably comprised between 3 and 10 mm.

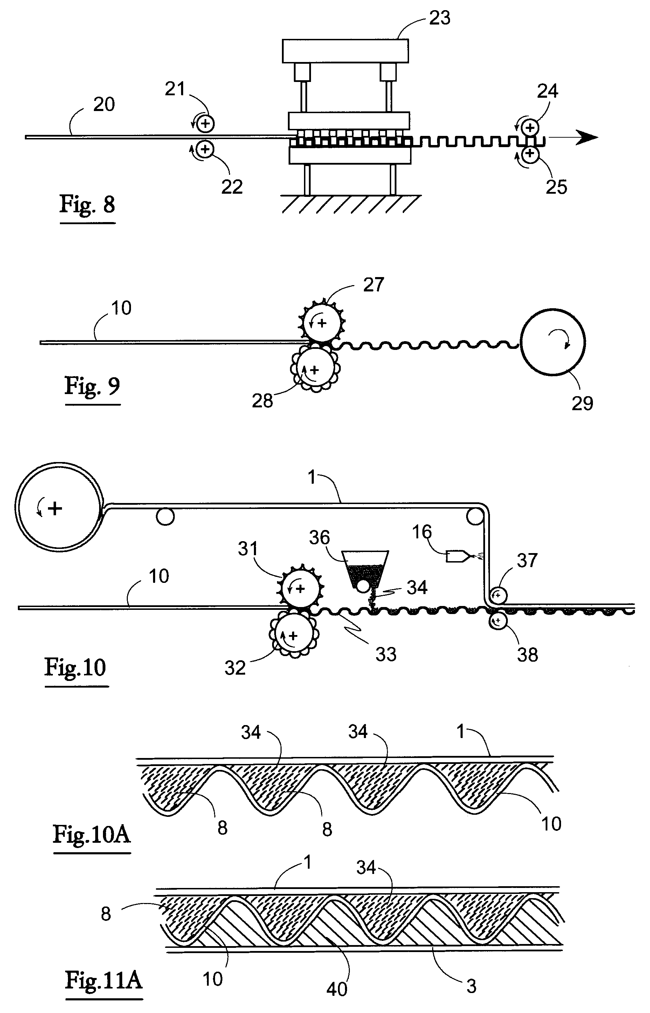

With reference to Figs. 8 to 11, different embodiments of the process according to the invention will be disclosed. In the Figures the absorbent structure according to the invention is formed as a continuous web from which the single absorbent cores are obtained by cutting.

Fig. 8 illustrates a way to realize an embossed web 10 forming the tridimensional layer. In accordance with this embodiment, a layer of fabric 20, driven by rolls 21, 22, is passed through a press 23 with cooperating molds disposed on both sides of the fabric, with the molds properly shaped to form a configuration of the above described type.

Another pair of drive rolls 24, 25 is provided at the output of the press 23. The temperature of the press is comprised between 0 and 180 °C.

According to the embodiment illustrated in Fig. 9, the layer or web of fabric 10 is drawn by a pair of rolls 27, 28 the surfaces of which are shaped for realizing the desired embossing pattern and the web is continuously relief printed as it passes through the rolls. The temperature of the embossing rolls is comprised between 0 and 180 °C, and the embossed product is wound to form a collecting roll 29.

The process illustrated in Fig. 10 shows the manufacture of a composite structure with SAP absorbent powders. The fabric 10 is drawn in the nip between a pair of rolls 31, 32 on the surfaces of which the embossing profiles are formed and the fabric is relief printed with recesses and reliefs. In the recesses of the embossed web 33 there are deposited powders 34 of the SAP type, from a dispenser 36. Then the web 33 is calendered together with a second fabric layer 1 on which a layer of hot melt adhesive has been applied by a dispenser 16. The two layers are then joined together when passing through a pair of calibrating rolls 37, 38 having smooth surfaces.

Fig. 10A shows the structure obtained through this process, wherein a space is indicated - formed by the containers 8 - that contains the absorbent material 34.

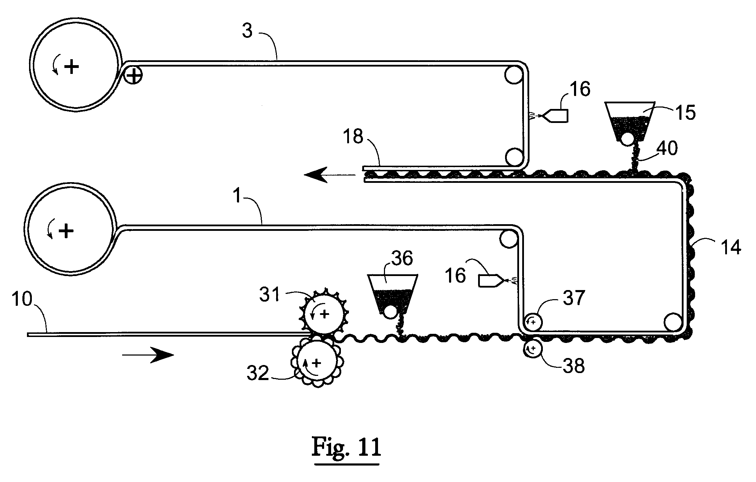

Finally, in the process shown in Fig. 11 using the same numeral references of Fig. 10, a web 14 substantially similar to the web produced in the apparatus of Fig. 10 is obtained, and this web 14 is subjected to a further deposition of a powder material 40 from a dispenser 15, and then to the application of another web 3, bonded through an adhesive supplied by a second dispenser 16. Fig. 11A shows the structure obtained through this process, wherein the spaces are indicated that contains the absorbent materials 34 and 40, respectively.

Le two structures shown in Figs. 10A and 11A constitute the absorbent core of an final absorbent article that can be manufactured either in line or out of line, at other times and/or in other plants.

While the invention has been disclosed with particular reference to preferred embodiments thereof, it is not to be limited to the illustrated embodiments, but includes all the obvious modifications and changes that will be evident to the skilled in the art.

## Claims

1. A layered absorbent structure, comprising two sheets (1, 3) of materials permeable to fluids and an absorbent core (2) sandwiched between them, characterized in that said absorbent core comprises a sheet of material (10) embossed with reliefs (4) so as to form recesses (6) separate from each other and projecting at least from a face of said sheet, said embossed sheet (10) being bonded to one (1) of said sheets at least along peripheral portions (5A) extending around the bases of said recesses (6), so as to form a tridimensional structure with separate and closed containers (8), at least partially filled with absorbent materials (11), and cavities or channels (7) adjacent and surrounding said containers (8), and capable of allowing an unhindered flow of fluid bypassing the containers (8) saturated by said fluid.

2. A structure as claimed in claim 1, characterized in that in said cavities or channels (7, 47) there is disposed a material (49) that does not hinder the flow of said flow of fluid.

3. A structure as claimed in claim 1 or 2, characterized in that the material in said closed containers (8) comprises a superabsorbent material (SAP) or a mixture of superabsorbent materials (11).

4. A structure as claimed in claim 1, 2 or 3, characterized in that the materials in said cavities or channels (7, 47) are selected from the group formed by substances capable of absorbing odours, scented substances, fluid-absorbent substances with a low capacity of absorption.

5. A structure as claimed in the preceding claims, characterized in that between one (1) of said sheets and said embossed layer (10) an ADL layer (59) is disposed.

6. A structure as claimed in the preceding claims, characterized in that said embossed layer comprises an ADL layer (69).

7. A structure as claimed in the preceding claims, characterized in that said absorbent material (11) is in form of powders, granules or fibers.

8. A process for manufacturing a layered absorbent structure as claimed in any of the preceding claims, characterized in that it comprises the following steps:
a. passing a sheet of web material (10) through means (23; 31, 32) capable of relief printing it into a tridimensional structure with recesses (6);
b. depositing a material (11, 34) in form of powders, granules or fibers inside at least some of said recesses;
c. deposing a sheet of material (1) over said tridimensional layer bonding them in corrispondence of the areas (5A) surrounding said recesses (6).

9. A process as claimed in claim 8, characterized in that it provides the applications of a further sheet (2) of a web material on the opposite side with respect to that on which said sheet (1) is applied.

10. A process as claimed in claim 9, characterized in that it provides the deposition of material (40; 49) in form of powders, granules or fibers inside at least some of the channels defined between said tridimensional sheet (10) and said further sheet (2).
